# EUROPEAN PATENT APPLICATION

(11) **EP 4 640 165 A1**
(43) Date of publication of application: **29.10.2025**
(21) Application number: 23905967.8
(22) Date of filing: 19.12.2023
(51) Int. Cl.: A61B 17/04, A61B 17/072

(54) **ELECTRIC SUTURING INSTRUMENT**

(30) Priority: 22.12.2022 CN 202211652057
(71) Applicant: B. J. Zh. F. Panther Medical Equipment Co., Ltd., Beijing 102200 (CN)
(72) Inventor: FAN, Hongli, Beijing 102200 (CN)
(74) Representative: Huang, Liwei
(86) International application number: PCT/CN2023/139995
(87) International publication number: WO 2024/131804

(57) **Abstract**

The present invention discloses an electric suturing apparatus, including a driving apparatus, a transmission apparatus, a bending apparatus and an actuator, a distal end of the driving apparatus is connected to a proximal end of the transmission apparatus, a distal end of the transmission apparatus is connected to a proximal end of the bending apparatus, and a distal end of the bending apparatus is connected to the actuator, the transmission apparatus includes a driving shaft, a bending propulsion apparatus, a bending control shaft; the driving apparatus includes a reduction motor, the reduction motor rotates forward and reversely, and drives the driving shaft to rotate forward and reversely, the rotation of the drive shaft drives the bending propulsion apparatus to make a linear reciprocating motion, and the bending propulsion apparatus drives the actuator to bend left and right through the bending apparatus. The electric suturing apparatus of the present invention is easy to operate and has a large bending angle, which has better technical effects.

## Description

### Technical Field

The present invention relates to the field of medical apparatus, and in particular relates to an electric suturing apparatus.

### Background technology

Laparoscopic surgery is a newly developed minimally invasive method, which is an inevitable trend in the development of future surgical methods. However, due to the limitation of space within the abdominal or thoracic cavity, minimally invasive surgical instruments are necessarily smaller and longer in order to achieve precise operation.

The most difficult part of minimally invasive laparoscopic surgery is undoubtedly the suturing. It requires the use of both hands to coordinate the suturing needle and the needle-holding instrument to make successive sutures in a limited space. Many surgeons find this maneuver extremely difficult. The exposure of the suturing needle can also lead to accidents, puncturing other organs of the patient or injuring the surgeon or nurse.

Conventional suturing apparatus for surgery, through the manipulation of the suturing apparatus on the steering knob control structure to manipulate the front actuator to the left or right rotation, usually formed by the left and right of the maximum 45 ° turn, the process requires two-handed operation, one hand to hold the instrument, the other hand to rotate the steering knob, and at the same time adjusted to the desired angle, you need to operate the other body to lock the position, the whole process is extremely inconvenient and time-consuming operation. The whole process is extremely inconvenient and time-consuming. Due to the differences in the patient's surgical site, the bending angle cannot fully satisfy the operation of the surgical site. Therefore, the suturing apparatus needs to be easy to operate and have a larger bending angle of the working part in order to meet the complex clinical needs.

### Contents of the invention

The present invention is to solve the technical problem in the prior art in which the operation of the suturing apparatus is complicated and the bending angle is insufficient.

To solve the above technical problems, the present invention provides the following technical solution:

An electric suturing apparatus, including a driving apparatus, a transmission apparatus, a bending apparatus and an actuator, a distal end of the driving apparatus is connected to a proximal end of the transmission apparatus, a distal end of the transmission apparatus is connected to a proximal end of the bending apparatus, and a distal end of the bending apparatus is connected to the actuator, wherein, the transmission apparatus includes a driving shaft, a bending propulsion apparatus, a bending control shaft; the driving apparatus includes a reduction motor, the reduction motor rotates forward and reversely, and drives the driving shaft to rotate forward and reversely, the rotation of the drive shaft drives the bending propulsion apparatus to make a linear reciprocating motion, and the bending propulsion apparatus drives the actuator to bend left and right through the bending apparatus.

Further, the reduction motor includes an output shaft, the output shaft is cooperatively connected with a drive shaft, the drive shaft includes a threaded rod, the threaded rod is cooperatively connected with a drive threaded hole provided in the bending propulsion apparatus; wherein the thread of the threaded rod is a self-locking thread.

Further, the bending control shaft is provided in an outer tube, and one end of the bending control shaft is connected to the bending propulsion apparatus, and the other end is connected to the bending apparatus; the bending apparatus is hinged to the outer tube to form a first vertical rotary axis, and hinged to the actuator to form a second vertical rotary axis.

Further, the bending apparatus specifically includes: a U-type bending pull piece, a bending support shaft, a bending joint, a bending piece connecting ring; the bending control shaft is fixedly connected to the U-type bending pull piece, and the two ends of the U-type bending pull piece pass through the bending joint and are connected to the bending piece connecting ring, and the bending piece connecting ring is fixedly connected to the actuator; the bending propulsion apparatus reciprocates in a straight line; the bending control shaft and the bending pull piece are driven to reciprocate together, thereby driving the actuator to rotate around the first vertical rotation axis and the second vertical rotation axis.

Further, the bending propulsion apparatus comprises: a first bending propulsion block, a second bending propulsion block, a pin; the first bending propulsion block, the second bending propulsion block are fixed by the pin to form a one-piece structure; the first bending propulsion block and the second bending propulsion block are able to be separated when the pin is removed. When the motor fails and cannot drive the actuator back to the initial position, and cannot safely exit the patient body, the pin can be removed to separate the first bending propulsion block and the second bending propulsion block, so that the second bending propulsion block can release the position locking state, and the actuator returns to the free state, and at this time, the apparatus can be safely exited from the patient body.

Further, the electric suturing apparatus comprises a position detection module, the position detection module is provided with a plurality of microswitches for controlling the position of the second bending propulsion block, thereby controlling the rotation angle of the actuator.

Further, the microswitch includes: a left limit microswitch, a zero position microswitch, a right limit microswitch, which correspond to a left bending limit position, a 0-degree position and a right bending limit position of the actuator, respectively.

Further, the electric suturing apparatus further comprises a bending button, and by stirring the bending button in a forward or reverse direction, the reduction motor is controlled to rotate in a forward or reverse direction, thereby controlling the actuator to turn in a left bending direction or a right bending direction.

Further, the electric suturing apparatus further comprises a power supply voltage detection module and/or a current detection module; the power supply voltage detection module comprises: a voltage detection IC and an alarm; the voltage detection IC detects in real time the power supply voltage of the motor driver IC after the power supply is supplied to the electric suturing apparatus, and the bending button is stirred to drive the reduction motor to rotate if the voltage meets the expected value, the alarm is started to alarm if the voltage does not meet an expected value, and a main microcontroller automatically suspends the subsequent drive operation; the current detection module includes a current detection IC, the current detection IC detects a drive current of the reduction motor during the bending process, and the reduction motor stops rotating when the drive current exceeds a threshold value. The power supply voltage detection module is set up to avoid transmission failure caused by insufficient torque output of the reduction motor due to insufficient voltage to ensure complete surgical operation. The current detection module can protect the operation safety of the electric suturing apparatus.

Further, an initialization operation is performed when the electric suturing apparatus is powered on, specifically: the reduction motor rotates forward until the second bending propulsion block triggers the left limit microswitch, and then stops rotating forward, at this time the actuator reaches the left bending limit, the reduction motor rotates reversely until a median protrusion provided on the second bending propulsion block triggers the zero position microswitch, an automatic delay stops rotating reversely, at this time the actuator reaches the 0-degree position.

With such a design, the present invention has at least the following advantages:
(1) The electric suturing apparatus of the present invention has a large bending angle, and both the left and right bending angles can reach 90, and it is infinitely rotatable, so that it can be arbitrarily stopped and locked in the current position during the bending process.
(2) The electric suturing apparatus of the present invention is easy to operate, and can be operated with one hand, reducing the time-consuming operation of instrument adjustment and saving surgical time.
(3) By setting a safety pin, the present invention can unlock the bending state of the front end after a malfunction, safely exit the patient's body, and ensure the safety of surgery.
(4) The present invention has a simple structure, good effect, low cost and easy process realization.

### Description of the accompanying drawings

The above is only an overview of the technical solution of the present invention, in order to be able to more clearly understand the technical means of the present invention, with the following accompanying drawings and specific embodiments of the present invention for further detailed description.
FIG. 1 is a schematic diagram of the decomposition of the components of the electric suturing apparatus of the present invention;
FIG. 2 is a schematic diagram of the decomposition of partial structure of the driving apparatus and transmission apparatus of the electric suturing apparatus of the present invention;
FIG. 3 is a schematic diagram of the structure of the bending piece connecting ring of the present invention;
FIG. 4 is a schematic diagram of the structure of the bending joint of the present invention;
FIG. 5 is a schematic diagram of an assembly section at the bending joint of the present invention;
FIG. 6 is a schematic diagram of the left bending of the electric suturing apparatus of the present invention;
FIG. 7 is a schematic diagram of the right bending of the electric suturing apparatus of the present invention;
FIG. 8 is a schematic diagram of position detection of the electric suturing apparatus of the present invention;
FIG. 9 is a logic diagram of the bending electric control of the electric suturing apparatus of the present invention.

### [Illustration of accompanying markings]

buffer rod component 1000, push-pull ring 1100, waist-shaped hole 1101, thread hole 1102, T-shaped hole 1102B, nut 1200, outer thread shaft 1201, first buffer spring 1300, first buffer rod 1400, first buffer rod thread shaft 1401, first buffer rod T-shaped shaft 1401B, first buffer rod base 1402, buffer sleeve 1500, sleeve thread hole A 1501, sleeve step hole 1502, sleeve thread hole B 1503, sleeve right cavity 1504, sleeve left cavity 1505, second buffer spring 1600, second buffer rod 1700, second buffer shaft step 1701, eccentric wheel component 2000, motor connection hole 2001, eccentric shaft 2002, first let-off slot 2003, second let-off slot 2004 , skeleton 3000, motor shaft output hole 3001, buffer rod part running hole 3002, motor fixing hole 3003, skeleton round hole 3004, microswitch mounting A hole 3005, microswitch mounting B hole 3006, drive motor 4000, motor output shaft 4001, fixing shaft 4002, electric control part 5000, first microswitch 5001, second microswitch 5002, a trigger button 5003, a power supply 5004, a main microcontroller 5005, a voltage detection IC 5006, a prompter 5007, a motor driver IC 5008, and a current detection IC 5009.

### Specific embodiments

The technical solutions in the embodiments of the present invention will be clearly and completely described below in conjunction with the accompanying drawings in the embodiments of the present invention, and it is clear that the described embodiments are only a part of the embodiments of the present invention and not all of the embodiments. Based on the embodiments in the present invention, all other embodiments obtained by a person of ordinary skill in the art without making creative labor are within the scope of protection of the present invention.

In this document, "up", "down" and the like are used only to indicate relative positional relationships between related parts, and not to limit the absolute positions of these related parts.

Each of the following parts is defined as a proximal end near the operator and a distal end away from the operator. This expression proximal end and distal end is used only to make the description simple and clear and should not be construed as any limitation of the present invention.

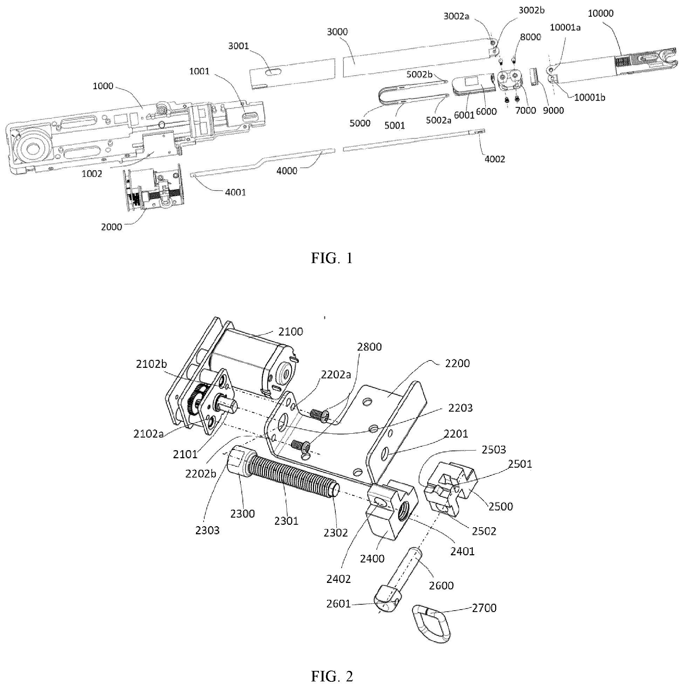

As shown in FIG. 1, an electric suturing apparatus, including a driving apparatus, a transmission apparatus, a bending apparatus and an actuator, a distal end of the driving apparatus is connected to a proximal end of the transmission apparatus, a distal end of the transmission apparatus is connected to a proximal end of the bending apparatus, and a distal end of the bending apparatus is connected to the actuator, wherein, the transmission apparatus includes a driving shaft 2300, a bending propulsion apparatus, a bending control shaft 4000; the driving apparatus includes a reduction motor 2100, the reduction motor 2100 rotates forward and reversely, and drives the driving shaft to rotate forward and reversely, the rotation of the drive shaft drives the bending propulsion apparatus to make a linear reciprocating motion, and the bending propulsion apparatus drives the actuator to bend left and right through the bending apparatus. As shown in FIG. 2, the reduction motor 2100 includes an output shaft 2101, the output shaft 2101 is cooperatively connected with a drive shaft 2300, the drive shaft 2300 includes a threaded rod 2301, the threaded rod 2301 is cooperatively connected with a drive threaded hole 2401 provided in the bending propulsion apparatus; wherein the thread of the threaded rod 2301 is a self-locking thread, and the lift angle of the thread is less than the equivalent friction angle of the helical pair, so as to enable the reduction motor 2100 to maintain the actuator's angular bending and self-locking when it stops at an arbitrary position and to realize the infinite rotational operation of the actuating structure 1000.

As shown in FIG. 2, the electric suturing apparatus further includes a bracket 2200, and the reduction motor 2100 is fixedly connected to the bracket 2200, specifically, the reduction motor 2100 is provided with fixing holes A 2102a, fixing holes B 2102b, which are fixedly connected to the bracket fixing holes A 2202a and bracket fixing holes B 2202b provided by the bracket 2200 by means of fixing screws. The bracket 2200 is provided with a main support hole 2203, and the output shaft 2101 of the reduction motor 2100 passes through the main support hole 2203 and is matchedly connected with the drive hole 2303 provided in the drive shaft 2300; the bracket 2200 is provided with a sub support shaft hole 2201, and the drive shaft 2300 is provided with a sub support shaft 2302, which is fitted into the sub support hole 2201.

As shown in FIG. 1, the electric suturing apparatus also includes a skeleton 1000 and an outer tube 3000; the driving apparatus 2000 is fitted into a cavity 1002 provided by the skeleton 1000; the outer tube 3000 is provided with a fixed waist-shaped hole 3001, connected to a tab 1001 provided by the skeleton 1000; the outer tube 3000 is fixed in a relatively fixed position relative to the driving apparatus 2000, and a bending control shaft 4000 is provided in the outer tube 3000.

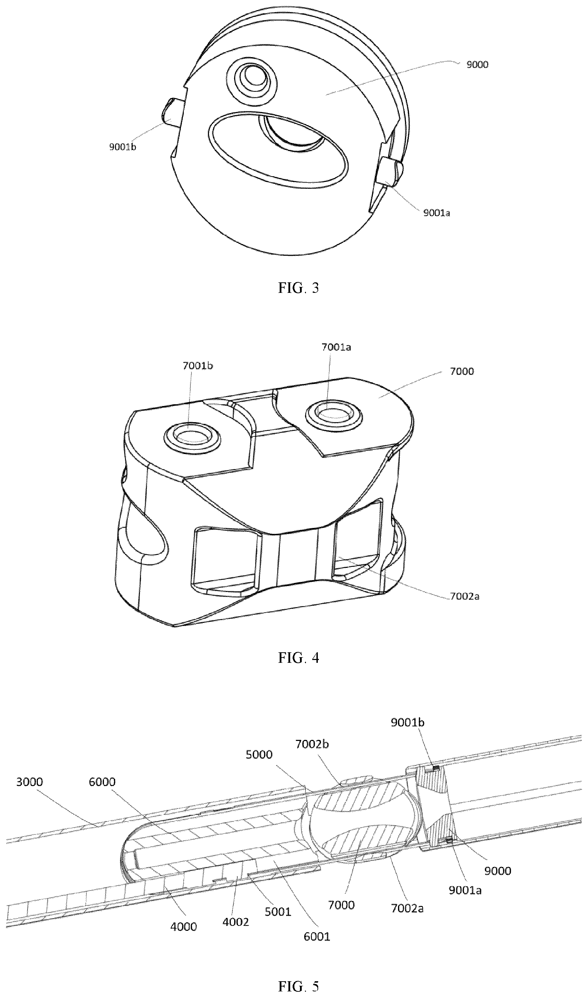

As shown in FIGS. 1 and 3-7, the bending control shaft 4000 is connected to the bending propulsion apparatus at one end and to the bending apparatus at the other end. Optionally, the bending control shaft 4000 is provided with a T-shaped shaft head 4001 connecting and matching with a T-shaped slot 2501 provided on the bending propulsion apparatus. The bending apparatus is hinged to the outer tube 3000 to form a first vertical rotation axis, and hinged to the actuator 1000 to form a second vertical rotation axis. The bending apparatus specifically includes: a U-shaped bending pull piece 5000, a bending support shaft 6000, a bending joint 7000, and a bending piece connecting ring 9000. the bending control shaft 4000 is provided with a waist-type tab 4002 and the bending pull piece 5000 is provided with a waist-type hole 5001 fixedly connected. The bending control shaft 4000 is placed in the semi-circular slot 6001 provided in the bending support shaft 6000, which serves as a fixed slide in the reciprocating movement of the bending control shaft 4000. The ends of the bending pull tabs 5000 pass through the bending joints 7000 and are connected to the bending tab connecting ring 9000. The two sides of the bending joint 7000 are respectively provided with right rectangular holes 7002a and left rectangular holes 7002b, and the two ends of the bending puller 5000 respectively pass through the right rectangular holes 7002a and left rectangular holes 7002b. The bending puller 5000 is provided with the right connecting holes 5002a and the left connecting holes 5002b, respectively, into the bending piece connecting ring 9000 provided with the right connecting pins 9001a and the left connecting pins 9001b. The bending tab connecting ring 9000 is fixedly connected to the actuator 10000. Four joint connection pins 8000 are provided, the first joint connection pin passes through the upper fixing position hole 3002a of the outer tube provided by the outer tube 3000, the upper joint connection hole 7001a provided by the bending joint 7000, and the second joint connection pin passes through the lower fixing position hole 3002b of the outer tube provided by the outer tube 3000, and the said upper joint connection hole 7001a, and the first joint connection pin and the second joint connection pin form a first vertical rotation axis. The rotation axis formed by the first joint connecting pin and the second joint connecting pin is a first vertical rotation axis; the third joint connecting pin passes through the upper connection hole 10001a provided by the actuator 10000, the lower connection hole 7001b provided by the bending joint 7000, and the fourth joint connecting pin passes through the lower connection hole 10001b provided by the actuator 10000, said lower connection hole 7001b, and the rotation axis formed by the third joint connecting pin and the fourth joint connecting pin is a second vertical rotation axis. The rotation axis formed by the third joint connecting pin and the fourth joint connecting pin is a second vertical rotation axis. The actuator 10000 can rotate around the axis of the joint connecting pin 8000 by the reciprocating movement of the bending propulsion apparatus, which drives the bending control axis 4000 and the bending pulling piece 5000 to reciprocate together. In this way, the actuator 10000 can achieve the purpose of left and right bending.

As shown in FIG. 2, the bending propulsion apparatus includes: a first bending propulsion block 2400, a second bending propulsion block 2500, and a pin 2600; the first bending propulsion block and the second bending propulsion block can be fixed as a one-piece structure by the pin 2600. The first bending propulsion block is provided with a protruding portion, the second bending propulsion block is provided with a recess, and the protruding portion can slide axially in the recess; when the pin 2600 is inserted into the first propulsion block circular hole 2502 provided in the second bending propulsion block 2500 and the first bending propulsion block 2402 provided in the first bending propulsion block 2400, the first bending propulsion block and the second bending propulsion block are fixed together; when the pin 2600 is pulled out 2600, the first bending propulsion block and the second bending propulsion block are separated. Preferably, a pull ring 2700 is also included, the pull ring 2700 being formed integrally through the 2601 pull ring hole provided in the safety pin 2600.

After the actuator 10000 has been bent during operation, if the motor malfunctions and it is not possible to drive the actuator 10000 back to the initial position, and it is not possible to safely exit the patient body, then the pull ring 2700 can be pulled so that the safety pin 2600 is detached from the circular hole 2402 provided in the first bending propulsion block 2400 and the circular hole 2502 provided in the second bending propulsion block 2500, and the first bending propulsion block and the second bending propulsion block can be detached. The first curvature propeller block and the second curvature propeller block can be separated, so that the second curvature propeller block 2500 is released from the position locking state, and the actuator 10000 is restored to the free state, and then the patient can be safely withdrawn from the patient's body.

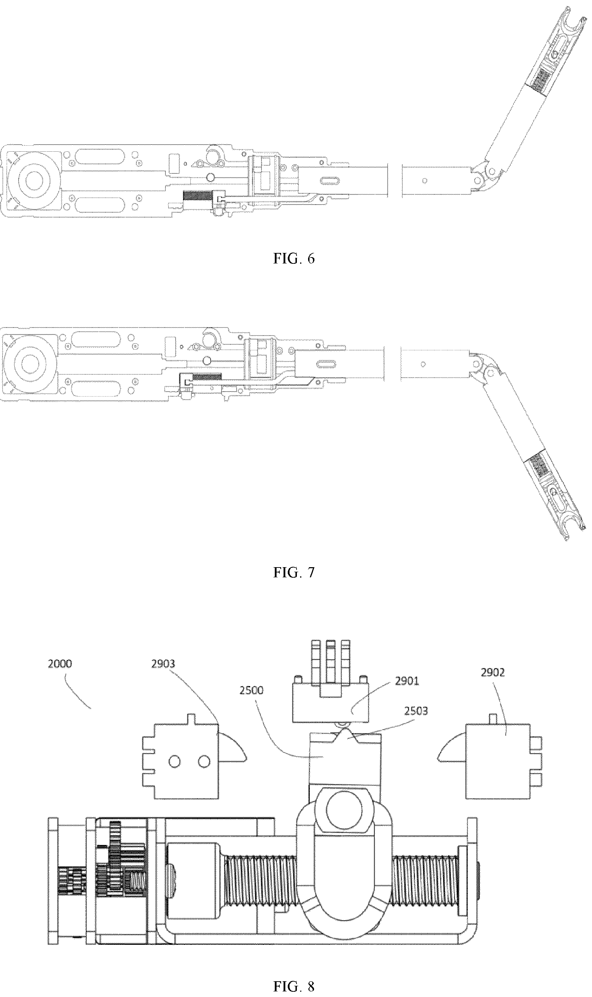

As shown in FIG. 8, the electric suturing apparatus further includes a bend button 11007 to control the reduction motor to rotate forward or reversely by toggling the bend button forward or reversely, thereby controlling the actuator to rotate in a left bend or a right bend. The electric suturing apparatus includes a position detection module, the position detection module a plurality of microswitches for controlling the position of the second bend-around propulsion block and thereby controlling the angle of rotation of the actuator structure 10000. Specifically, the microswitches include a zero microswitch 2901, a left limit microswitch 2902, and a right limit microswitch 2903. the reduction motor 2100 rotates forward to the point where the second bending propulsion block 2500 triggers the left limit microswitch 2902, the reduction motor automatically stops rotating forward, at which time the actuator 10000 is in the left bending limit, and the reduction motor 2100 rotates in the reverse direction to the point where the When the zero position microswitch 2901 is triggered by the center tab 2503 provided in the second bending propulsion block 2500, the reduction motor automatically stops rotating in the reverse direction after a time delay, and at this time, the actuator 10000 is in the 0-degree position. The reduction motor 2100 again rotates in the reverse direction until the second bending propulsion block 2500 triggers the right limit microswitch 2903 the reduction motor automatically stops rotating in the reverse direction, at which time the actuator 10000 is in the right bending limit position. Wherein, the actuator has a bending angle of 90 degrees at the left bending limit state and the right bending limit state.

The electric sewing apparatus includes a power supply voltage detection module, and said power supply voltage detection module includes: a voltage detection IC and an alarm; after the power supply is supplied to the electric sewing apparatus, the voltage detection IC detects the power supply voltage of the motor driver IC in real time, and if the voltage meets the expected value, the bending button is toggled to drive the reduction motor to rotate, and if the voltage does not meet the expected value, the alarm is activated for alarming, and the main microcontroller automatically suspends the subsequent If the voltage does not meet the expected value, the alarm will be activated and the main microcontroller will automatically suspend the subsequent driving operation. Thus, it can avoid the transmission failure caused by insufficient torque output of the reduction motor 2100 due to insufficient voltage, and ensure the complete surgical operation.

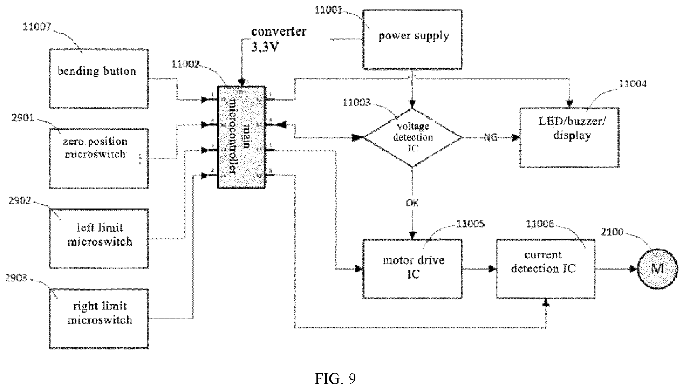

As shown in Figure 9, the suturing apparatus is connected to the power supply 11001 converted to 3.3V for the main microcontroller 11002 power supply, and preset procedures through the voltage detection IC 11003 real-time detection of the voltage supply for the motor driver IC 11005, the voltage is in line with the expected value, and then toggle the 11007 bending key 2100 reduction motor rotation, if the voltage is not in line with the expected value of the design of alarms 11004 will be If the voltage does not meet the expected design value alarm 11004 will prompt a warning through the LED/buzzer/display, and the main microcontroller 11002 automatically suspends the subsequent drive operation, avoiding insufficient voltage to cause insufficient torque output of the reduction motor 2100 to appear transmission faults, and ensuring that the surgical operation is complete.

In addition, the motorized suturing apparatus also includes a current detection IC 11006, which detects the drive current of the reduction motor 2100 through the current detection IC 11006 during the bending process, and stops the reduction motor 2100 from rotating when it exceeds the threshold value, so as to protect the safety of the operation of the instrument.

When the electric suturing apparatus is connected to the power supply, the initialization operation is carried out to ensure that the actuator is located in the initial position of 0-degree; specifically, when the apparatus is initially used, the reduction motor 2100 rotates forward until the second bending propulsion block 2500 triggers the left limit microswitch 2902, and then the motor automatically stops rotating in the forward direction, at this time, the actuator 10000 reaches the limit of the left bending, and the reduction motor 2100 rotates reversely, until the center tab provided on the second bending propulsion block 2500 triggers the zero position microswitch 2901; with an automatic delay, the motor stops rotating in the reverse direction, at which time the actuator 10000 is in the initial position of 0-degree. In the subsequent operation of the forward toggle turn button 11007 deceleration motor 2100 forward rotation, triggering the left limit microswitch 2902 that stops rotating, the forward toggle turn button 11007 temporarily disabled. The reverse toggle turn button 11007 reduces the rotation of the motor 2100 in the reverse direction, triggers the right limit microswitch 2903 to stop the rotation, and the reverse toggle turn button 11007 is temporarily deactivated.

Other embodiments of the invention will readily come to mind by those skilled in the art upon consideration of the invention disclosed in the specification and embodiments. This application is intended to cover any variations, uses, or adaptations of the present invention that follow the general principles of the invention and include common knowledge or customary technical means in the art not disclosed herein. The specification and embodiments are to be regarded as exemplary only, and the true scope and spirit of the invention is indicated by the following claims.

It should be understood that the present invention is not limited to the precise structure which has been described above and illustrated in the accompanying drawings, and that various modifications and alterations may be made without departing from its scope. The scope of the invention is limited only by the appended claims.

## Claims

1. An electric suturing apparatus, including a driving apparatus, a transmission apparatus, a bending apparatus and an actuator, a distal end of the driving apparatus is connected to a proximal end of the transmission apparatus, a distal end of the transmission apparatus is connected to a proximal end of the bending apparatus, and a distal end of the bending apparatus is connected to the actuator, **characterized in that** the transmission apparatus includes a driving shaft, a bending propulsion apparatus, a bending control shaft; the driving apparatus includes a reduction motor, the reduction motor rotates forward and reversely, and drives the driving shaft to rotate forward and reversely, the rotation of the drive shaft drives the bending propulsion apparatus to make a linear reciprocating motion, and the bending propulsion apparatus drives the actuator to bend left and right through the bending apparatus.

2. The electric suturing apparatus as claimed in claim 1, **characterized in that** the reduction motor includes an output shaft, the output shaft is cooperatively connected with a drive shaft, the drive shaft includes a threaded rod, the threaded rod is cooperatively connected with a drive threaded hole provided in the bending propulsion apparatus; wherein the thread of the threaded rod is a self-locking thread.

3. The electric suturing apparatus as claimed in claim 2, **characterized in that** the bending control shaft is provided in an outer tube, and one end of the bending control shaft is connected to the bending propulsion apparatus, and the other end is connected to the bending apparatus; the bending apparatus is hinged to the outer tube to form a first vertical rotary axis, and hinged to the actuator to form a second vertical rotary axis.

4. The electric suturing apparatus as claimed in claim 3, **characterized in that** the bending apparatus specifically includes: a U-type bending pull piece, a bending support shaft, a bending joint, a bending piece connecting ring; the bending control shaft is fixedly connected to the U-type bending pull piece, and the two ends of the U-type bending pull piece pass through the bending joint and are connected to the bending piece connecting ring, and the bending piece connecting ring is fixedly connected to the actuator; the bending propulsion apparatus reciprocates in a straight line; the bending control shaft and the bending pull piece are driven to reciprocate together, thereby driving the actuator to rotate around the first vertical rotation axis and the second vertical rotation axis.

5. The electric suturing apparatus as claimed in claim 4, **characterized in that** the bending propulsion apparatus comprises: a first bending propulsion block, a second bending propulsion block, a pin; the first bending propulsion block, the second bending propulsion block are fixed by the pin to form a one-piece structure; the first bending propulsion block and the second bending propulsion block are able to be separated when the pin is removed.

6. The electric suturing apparatus as claimed in claim 5, **characterized in that** the electric suturing apparatus comprises a position detection module, the position detection module is provided with a plurality of microswitches for controlling the position of the second bending propulsion block, thereby controlling the rotation angle of the actuator.

7. The electric suturing apparatus as claimed in claim 6, **characterized in that** the microswitch includes: a left limit microswitch, a zero position microswitch, a right limit microswitch, which correspond to a left bending limit position, a 0-degree position and a right bending limit position of the actuator, respectively.

8. The electric suturing apparatus as claimed in any one of claims 1-7, **characterized in that** it further comprises a bending button, and by stirring the bending button in a forward or reverse direction, the reduction motor is controlled to rotate in a forward or reverse direction, thereby controlling the actuator to turn in a left bending direction or a right bending direction.

9. The electric suturing apparatus as claimed in any one of claims 1-7, **characterized in that** the electric suturing apparatus further comprises a power supply voltage detection module and/or a current detection module; the power supply voltage detection module comprises: a voltage detection IC and an alarm; the voltage detection IC detects in real time the power supply voltage of the motor driver IC after the power supply is supplied to the electric suturing apparatus, and the bending button is stirred to drive the reduction motor to rotate if the voltage meets the expected value, the alarm is started to alarm if the voltage does not meet an expected value, and a main microcontroller automatically suspends the subsequent drive operation; the current detection module includes a current detection IC, the current detection IC detects a drive current of the reduction motor during the bending process, and the reduction motor stops rotating when the drive current exceeds a threshold value.

10. The electric suturing apparatus as claimed in claim 7, **characterized in that** an initialization operation is performed when the electric suturing apparatus is powered on, specifically: the reduction motor rotates forward until the second bending propulsion block triggers the left limit microswitch, and then stops rotating forward, at this time the actuator reaches the left bending limit, the reduction motor rotates reversely until a median protrusion provided on the second bending propulsion block triggers the zero position microswitch, an automatic delay stops rotating reversely, at this time the actuator reaches the 0-degree position.
